# EUROPEAN PATENT APPLICATION

(11) **EP 4 541 417 A1**
(43) Date of publication of application: **23.04.2025**
(21) Application number: 24191475.3
(22) Date of filing: 29.07.2024
(51) Int. Cl.: A61N 5/06

(54) **FACIAL IRRADIATION DEVICE**

(30) Priority: 20.10.2023 US 202318490802
(71) Applicant: Currentbody.com Limited, Cheadle Hulme SK8 6QH (GB)
(72) Inventor: WRENSHALL, Emily, Cheadle Hulme, SK8 6QH (GB)
(74) Representative: Valet Patent Services Limited

(57) **Abstract**

A facial irradiation device includes a facial mask including an outer layer, a flexible Printed Circuit Board (PCB) attached to the outer layer, a plurality of irradiation sources attached to the flexible PCB, and an inner layer attached to the flexible PCB and/or the outer layer. Furthermore, the facial irradiation device includes a power source configured to provide electrical power. Also, the facial irradiation device includes a control module comprising a processor and a memory unit operably connected to the processor. The memory unit comprises machine-readable instructions that when executed by the processor, enable the processor to control supply of the electrical power to the plurality of irradiation sources, such that, the plurality of irradiation sources is configured to be operated in a plurality of operating modes.

## Description

### TECHNICAL FIELD

The present invention generally relates to therapeutic devices. More specifically the present invention relates to light or electromagnetic radiation-based therapeutic devices.

### BACKGROUND ART

Light therapy devices have been known in the art for quite a while. Many such devices are designed to be wearable in nature such as in the shape of a face mask. The aim of such devices is to impart irradiation to a facial region of a user to cause skin regeneration and repair as the skin absorbs photons from the irradiation and enhances the production of Adenosine triphosphate (ATP) and collagen. Such devices in construction generally have an outer layer and a transparent inner layer and several LEDs located between the outer layer and the transparent inner layer. The LEDs have been located in such a manner that the LEDs are directed toward the inner transparent layer. In many such devices, the LEDs, in whole or in part, may be replaced with other irradiation sources such as, but not limited to, halogen lamps or LASERs.

In general, irradiation sources, either LEDs or other irradiation sources such as halogen lamps and LASERs, emit radiation in red or infrared wavelengths of the electromagnetic spectrum. Moreover, the LED with the red and the infrared wavelengths are generally placed adjacent to each other in pairs, or dual-mode LEDs are used as the irradiation sources to provide alternating pulses of the red and the infrared wavelengths. However, little effort is made to design distribution patterns of the irradiation sources to cater to the disparate needs of different regions of facial regions of the user. Moreover, the conventional face masks generally operate in a maximum of two modes, wherein in the first mode all the irradiation sources are simultaneously activated and in the second mode the red and the infrared irradiation sources are alternatively activated.

Therefore, there is a need for a facial irradiation device that overcomes the disadvantages and limitations associated with the prior art and provides a more satisfactory solution.

### OBJECTS OF THE INVENTION

Some of the objects of the invention are as follows:

An object of the present invention is to provide a facial irradiation device that includes a distribution pattern for irradiation sources that caters to the varied needs of several different regions of a facial region of the user.

Another object of the present invention is to provide a facial irradiation device that can be operated in several modes to facilitate several kinds of recreational and therapeutic applications.

Another object of the present invention is to provide a facial irradiation device that uses irradiation sources capable of emitting electromagnetic radiation of infrared, red, deep red, green, and yellow wavelengths.

It is also an object of the present invention to provide a facial irradiation device that uses electrical power received from batteries installed in a control module, thereby making the facial irradiation device compact and portable.

### SUMMARY OF THE INVENTION

According to a first aspect of the present invention, there is provided a facial irradiation device. The facial irradiation device includes a facial mask including an outer layer that includes a first surface facing towards the ambient and a second surface facing opposite to the first surface. The facial mask further includes a flexible Printed Circuit Board (PCB) attached to the outer layer. The flexible PCB includes a third surface facing towards the second surface and a fourth surface facing opposite to the third surface. Further, the facial mask includes a plurality of irradiation sources attached to the flexible PCB at the fourth surface. The facial mask further includes an inner layer attached to the flexible PCB and/or the outer layer. The inner layer includes a fifth surface facing toward the fourth surface and a sixth surface configured to face towards a facial region of a user. Furthermore, the facial irradiation device includes a power source configured to provide electrical power. Also, the facial irradiation device includes a control module comprising a processor and a memory unit operably connected to the processor. The memory unit includes machine-readable instructions that when executed by the processor, enable the processor to control supply of the electrical power to the plurality of irradiation sources, such that, the plurality of irradiation sources is configured to be operated in a plurality of operating modes.

In one embodiment of the invention, the plurality of irradiation sources is selected from a group consisting of Light Emitting Diodes (LEDs) and lasers.

In one embodiment of the invention, the plurality of irradiation sources is configured to emit electromagnetic radiation in wavelengths of ranges comprising 525 - 540 nm, 585 - 595 nm, 625 - 640 nm, 655 - 665 nm, and 825 - 835 nm.

In one embodiment of the invention, the plurality of irradiation sources is attached to the fourth surface in accordance with a predetermined distribution pattern including a plurality of irradiation zones.

In one embodiment of the invention, for operating the plurality of irradiation sources in an operating mode of the plurality of operating modes, the processor is further enabled to receive a control input corresponding to the operating mode, from a user computing device, via a communication interface of the control module, the control input comprising a wavelength assignment scheme, the wavelength assignment scheme comprising a plurality of respective wavelengths assigned to the plurality of irradiation zones.

In one embodiment of the invention, the plurality of irradiation zones is divided into a first group of irradiation zones, a second group of irradiation zones, and a third group of irradiation zones, such that, the first group of irradiation zones is configured to be around a forehead region and a mouth region of the facial region, the second group of irradiation zones is configured to be around two eyes of the facial region, and the third group of irradiation zones is configured to be around a nose region, a left side-cheek, a right side-cheek, and a jaw region of the facial region.

In one embodiment of the invention, irradiation sources in the first group of irradiation zones are configured to emit electromagnetic radiation of wavelengths 625 - 640 nm, 655 - 665 nm, and 825 - 835 nm, irradiation sources in the second group of irradiation zones are configured to emit electromagnetic radiation of wavelengths 525 - 540 nm, and irradiation sources in the third group of irradiation zones are configured to emit electromagnetic radiation of wavelengths 585 - 595 nm.

In one embodiment of the invention, the plurality of operating modes includes a first operating mode wherein the irradiation sources in the first group of irradiation zones are activated to emit the electromagnetic radiation with the wavelengths of 625 - 640 nm, 655 - 665 nm, and 825 - 835 nm. The plurality of operating modes further includes a second operating mode wherein the irradiation sources in the first group of irradiation zones are activated to emit the electromagnetic radiation with the wavelengths of 825 - 835 nm and the irradiation sources in the second group of irradiation zones are activated to emit the electromagnetic radiation with the wavelengths of 525 - 540 nm. Furthermore, the plurality of operating modes includes a third operating mode wherein the irradiation sources in the third group of irradiation zones are activated to emit the electromagnetic radiation with the wavelengths of 585 - 595 nm. Also, the plurality of operating modes includes a fourth operating mode wherein the irradiation sources in the first group of irradiation zones are activated to emit the electromagnetic radiation with the wavelengths of 625 - 640 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

In one embodiment of the invention, the plurality of operating modes comprises a fifth operating mode wherein the irradiation sources in the first group of irradiation zones are activated to emit the electromagnetic radiation with the wavelengths of 625 - 640 nm, 655 - 665 nm, and 825 - 835 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

In one embodiment of the invention, the processor is further enabled to switch between the plurality of operating modes based on a control input received from a user computing device via a communication interface of the control module.

In one embodiment of the invention, the power source includes one or more rechargeable batteries and the control module includes an indicator configured to indicate the remaining capacity of the power source while in use.

In one embodiment of the invention, the second surface includes a plurality of protrusions configured to be in contact with the facial region of the user to maintain a predetermined gap between the inner layer and the facial region of the user.

In one embodiment of the invention, the inner layer is made up of a transparent silicone material.

According to a second aspect of the present invention, there is provided a facial irradiation device. The facial irradiation device includes a face mask. The face mask includes an outer layer including a first surface facing towards the ambient and a second surface facing opposite to the first surface. The face mask further includes a flexible Printed Circuit Board (PCB) attached to the outer layer. The flexible PCB includes a third surface facing towards the second surface and a fourth surface facing opposite to the third surface. The face mask further includes a plurality of irradiation sources attached to the flexible PCB at the fourth surface. Also, the face mask includes an inner layer attached to the flexible PCB and/or the outer layer, the inner layer comprising a fifth surface facing towards the fourth surface and a sixth surface configured to be facing towards a facial region of a user. The facial irradiation device further includes a power source configured to provide electrical power. Also, the facial irradiation device includes a control module including a processor and a memory unit operably connected to the processor. The memory unit includes machine-readable instructions that when executed by the processor, enable the processor to control supply of the electrical power to the plurality of irradiation sources, such that, the plurality of irradiation sources is configured to be operated in a plurality of operating modes. The plurality of irradiation sources is attached to the fourth surface in accordance with a predetermined distribution pattern comprising a plurality of irradiation zones. Also, for operating the plurality of irradiation sources in an operating mode of the plurality of operating modes, the processor is further enabled to receive a control input corresponding to the operating mode, from a user computing device via a communication interface of the control module. The control input includes a wavelength assignment scheme. The wavelength assignment scheme includes a plurality of respective wavelengths assigned to the plurality of irradiation zones.

According to a third aspect of the present invention, there is provided a method for operating a facial irradiation device in an operating mode of a plurality of operating modes. The method includes providing a plurality of irradiation sources on a flexible Printed Circuit Board (PCB) in accordance with a predetermined distribution pattern. The predetermined distribution pattern includes a plurality of irradiation zones. The method further includes receiving, by a processor of a control module, a control input corresponding to the operating mode, from a user computing device via a communication interface of the control module. The control input includes a wavelength assignment scheme. The wavelength assignment scheme includes a plurality of respective wavelengths assigned to the plurality of irradiation zones. Also, the method includes controlling, by the processor, supply of electrical power, from a power source to the plurality of irradiation sources based on the wavelength assignment scheme.

In one embodiment of the invention, the method further includes dividing the plurality of irradiation zones into a first group of irradiation zones, a second group of irradiation zones, and a third group of irradiation zones, such that, the first group of irradiation zones is located around a forehead region and a mouth region of the facial region, the second group of irradiation zones is located around two eyes of the facial region, and the third group of irradiation zones is located around a nose region, a left side-cheek, a right side-cheek, and a jaw region of the facial region.

In one embodiment of the invention, the method further includes configuring irradiation sources in the first group of irradiation zones to emit electromagnetic radiation of wavelengths 625 - 640 nm, 655 - 665 nm, and 825 - 835 nm, configuring irradiation sources in the second group of irradiation zones to emit electromagnetic radiation of wavelengths 525 - 540 nm, and configuring irradiation sources in the third group of irradiation zones to emit electromagnetic radiation of wavelengths 585 - 595 nm.

In one embodiment of the invention, the method further includes switching, by the processor, between the plurality of operating modes based on the control input received from a user computing device via a communication interface of the control module

In the context of the specification, the term "processor" refers to one or more of a microprocessor, a microcontroller, a general-purpose processor, a Field Programmable Gate Array (FPGA) or an Application Specific Integrated Circuit (ASIC), and the like.

In the context of the specification, the phrase "memory unit" refers to volatile storage memory, such as Static Random Access Memory (SRAM) and Dynamic Random Access Memory (DRAM) of types such as Asynchronous DRAM, Synchronous DRAM, Double Data Rate SDRAM, Rambus DRAM, and Cache DRAM, etc.

In the context of the specification, the phrase "storage device" refers to a non-volatile storage memory such as EPROM, EEPROM, flash memory, or the like.

In the context of the specification, the phrase "communication interface" refers to a device or a module enabling direct connectivity via wires and connectors such as USB, HDMI, VGA, or wireless connectivity such as Bluetooth or Wi-Fi, or Local Area Network (LAN) or Wide Area Network (WAN) implemented through TCP/IP, IEEE 802.x, GSM, CDMA, LTE, or other equivalent protocols.

In the context of this specification, terms like "light", "radiation", "irradiation", "emission" and "illumination", etc. refer to electromagnetic radiation in frequency ranges varying from the Ultraviolet (UV) frequencies to Infrared (IR) frequencies and wavelengths, wherein the range is inclusive of UV and IR frequencies and wavelengths. It is to be noted here that UV radiation can be categorized in several manners depending on respective wavelength ranges, all of which are envisaged to be under the scope of this invention. For example, UV radiation can be categorized as, Hydrogen Lyman-α (122-121 nm), Far UV (200-122 nm), Middle UV (300-200 nm), Near UV (400-300 nm). The UV radiation may also be categorized as UVA (400-315 nm), UVB (315-280 nm), and UVC (280-100 nm) Similarly, IR radiation may also be categorized into several categories according to respective wavelength ranges which are again envisaged to be within the scope of this invention. A commonly used subdivision scheme for IR radiation includes Near IR (0.75-1.4 µm), Short-Wavelength IR (1.4-3 µm), Mid-Wavelength IR (3-8 µm), Long-Wavelength IR (8-15 µm) and Far IR (15-1000 µm).

In the context of the specification, a "polymer" is a material made up of long chains of organic molecules (having eight or more organic molecules) including, but not limited to, carbon, nitrogen, oxygen, and hydrogen as their constituent elements. The term polymer is envisaged to include both naturally occurring polymers such as wool, and synthetic polymers such as polyethylene and nylon.

In the context of the specification, "Light Emitting Diodes (LEDs)" refer to semiconductor diodes capable of emitting electromagnetic radiation when supplied with an electric current. The LED are characterized by their superior power efficiencies, smaller sizes, rapidity in switching, physical robustness, and longevity when compared with incandescent or fluorescent lamps. In that regard, the plurality of LEDs may be through-hole type LEDs (generally used to produce electromagnetic radiations of red, green, yellow, blue and white colors), Surface Mount Technology (SMT) LEDs, Bi-color LEDs, Pulse Width Modulated RGB (Red-Green-Blue) LEDs, and high-power LEDs, etc.

Materials used in the one or more LEDs may vary from one embodiment to another depending upon the frequency of radiation required. Different frequencies can be obtained from LEDs made from pure or doped semiconductor materials. Commonly used semiconductor materials include nitrides of Silicon, Gallium, Aluminum, and Boron, and Zinc Selenide, etc. in pure form or doped with elements such as Aluminum and Indium, etc. For example, red and amber colors are produced from Aluminum Indium Gallium Phosphide (AlGaInP) based compositions, while blue, green, and cyan use Indium Gallium Nitride based compositions. White light may be produced by mixing red, green, and blue lights in equal proportions, while varying proportions may be used for generating a wider color gamut. White and other colored lightings may also be produced using phosphor coatings such as Yttrium Aluminum Garnet (YAG) in combination with a blue LED to generate white light and Magnesium doped potassium fluorosilicate in combination with blue LED to generate red light. Additionally, near Ultraviolet (UV) LEDs may be combined with europium-based phosphors to generate red and blue lights and copper and zinc doped zinc sulfide-based phosphor to generate green light.

In addition to conventional mineral-based LEDs, one or more LEDs may also be provided on an Organic LED (OLED) based flexible panel or an inorganic LED-based flexible panel. Such OLED panels may be generated by depositing organic semiconducting materials over Thin Film Transistor (TFT) based substrates. Further, discussion on generation of OLED panels can be found in Bardsley, J. N (2004), "International OLED Technology Roadmap", IEEE Journal of Selected Topics in Quantum Electronics, Vol. 10, No. 1, that is included herein in its entirety, by reference. An exemplary description of flexible inorganic light-emitting diode strips can be found in granted U.S. Pat. No. 7,476,557 B2, titled "Roll-to-roll fabricated light sheet and encapsulated semiconductor circuit devices", which is included herein in its entirety, by reference.

In several embodiments, the one or more LEDs may also be micro-LEDs described through U.S. Pat. Nos. 8,809,126 B2, 8,846,457 B2, 8,852,467 B2, 8,415,879 B2, 8,877,101 B2, 9,018,833 B2 and their respective family members, assigned to NthDegree Technologies Worldwide Inc., which are included herein by reference, in their entirety. The one or more LEDs, in that regard, may be provided as a printable composition of the micro-LEDs, printed on a substrate.

### BRIEF DESCRIPTION OF THE ACCOMPANYING DRAWINGS

The accompanying drawings illustrate the best mode for carrying out the invention as presently contemplated and set forth hereinafter. The present invention may be more clearly understood from a consideration of the following detailed description of the preferred embodiments taken in conjunction with the accompanying drawings wherein like reference letters and numerals indicate the corresponding parts in various figures in the accompanying drawings, and in which:
FIG. 1 illustrates an exploded view of a facial mask of a facial irradiation device, in accordance with an embodiment of the present invention;
FIG. 2 illustrates a predetermined distribution pattern of a plurality of irradiation sources, in accordance with an embodiment;
FIG. 3 illustrates a connection between a control module and the facial mask of the facial irradiation device, in accordance with an embodiment of the present invention
FIG. 4 illustrates an example user interface of the user computing device for generating the wavelength assignment scheme, in accordance with an embodiment of the present invention; and
FIG. 5 illustrates a method for operating the facial irradiation device in an operating mode of the plurality of operating modes, in accordance with an embodiment of the present invention.

### DETAILED DESCRIPTION

Embodiments of the present invention disclosure will be described more fully hereinafter with reference to the accompanying drawings in which like numerals represent like elements throughout the figures, and in which example embodiments are shown.

The detailed description and the accompanying drawings illustrate the specific exemplary embodiments by which the disclosure may be practiced. These embodiments are described in detail to enable those skilled in the art to practice the invention illustrated in the disclosure. It is to be understood that other embodiments may be utilized, and other changes may be made, without departing from the spirit or scope of the present disclosure. The following detailed description is therefore not to be taken in a limiting sense, and the scope of the present invention disclosure is defined by the appended claims. Embodiments of the claims may, however, be embodied in many different forms and should not be construed as limited to the embodiments set forth herein.

Embodiments of the present invention provide a facial irradiation device including a facial mask and a control module. The facial mask includes several irradiation sources such as Light Emitting Diodes (LEDs), lasers, lamps, etc. that are capable of emitting electromagnetic radiation of varied wavelengths, such as Infrared, Red, Deep Red, Yellow, and Green. The varied wavelengths may be applied to various regions of a facial region of a user to achieve optimal results of skin rejuvenation and repair. The facial irradiation device is further envisaged to operate in several operating modes suited for several different kinds of therapies and for several different skin types.

The facial irradiation device is envisaged to be powered by rechargeable batteries making the facial irradiation device compact and portable. The batteries may be located in the control module. Furthermore, the control module may be provided with a processor and a memory unit for the configuration of several different modes of the facial irradiation device, and for receiving control inputs from a user computing device, in a manner that the processor can reconfigure the facial irradiation device following the received control inputs.

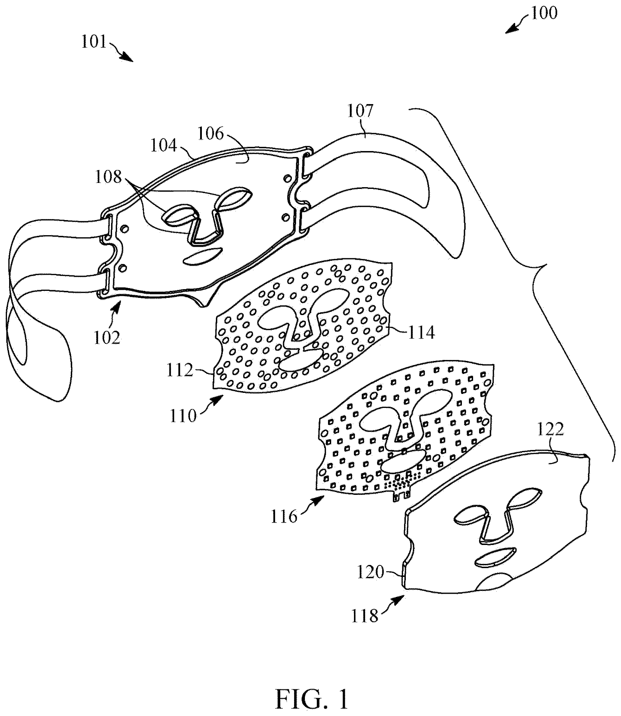

Several embodiments of the present invention will now be discussed in detail taking **FIGS 1-5** as reference.

**FIG. 1** illustrates an exploded view of a facial mask **101** of a facial irradiation device **100**, in accordance with an embodiment of the present invention. The face mask **101** includes an outer layer **102**, a flexible Printed Circuit Board (PCB) **110**, and an inner layer **118** arranged in a direction moving from ambient towards the facial region of a user. The outer layer **102** includes a first surface **104** facing towards the ambient and a second surface **106** facing opposite to the first surface **104**. A strap **107** made up of a flexible material such as fabric or leather is attached at two sides of the outer layer **102**. The strap **107** facilitates fastening of the face mask **101** to the facial region of the user. Furthermore, the second surface **106** includes a plurality of protrusions **108** provided near cutouts for the eyes and nose of the user. The cutouts for the eyes and the nose allow the user to see the ambient and breathe comfortably during the usage of the face mask **101**. The plurality of protrusions **108** are configured to be in contact with the facial region of the user to maintain a predetermined gap between the inner layer **118** and the facial region of the user. In several example embodiments, the predetermined gap may vary between 0.5 cm to 3 cm. The inner layer **118** is envisaged to be made up of a transparent silicone material. Further, the inner layer **118** is attached to the flexible PCB **110** and/or the outer layer **102**. The inner layer **118** includes a fifth surface **120** facing toward the fourth surface **114**, and a sixth surface **122** configured to be facing toward the facial region of a user.

The flexible PCB **110** is attached to the outer layer **102**. The flexible PCB **110** includes a third surface **112** facing towards the second surface **106**, and a fourth surface **114** facing opposite to the third surface **112**. A plurality of irradiation sources **116** have been attached to the flexible PCB **110** at the fourth surface **114**. In several embodiments of the invention, the plurality of irradiation sources **116** may be selected from a group consisting of Light Emitting Diodes (LEDs), lasers, lamps, or any other equivalent sources of electromagnetic radiation. In several embodiments of the invention, the plurality of irradiation sources **116** is configured to emit electromagnetic radiation in wavelengths of ranges comprising 525 - 540 nm (Green), 585 - 595 nm (Yellow), 625 - 640 nm (Red), 655 - 665 nm (Deep Red), and 825 - 835 nm (Infrared). In several embodiments of the invention, the plurality of irradiation sources **116** is attached to the fourth surface **114** in accordance with a predetermined distribution pattern.

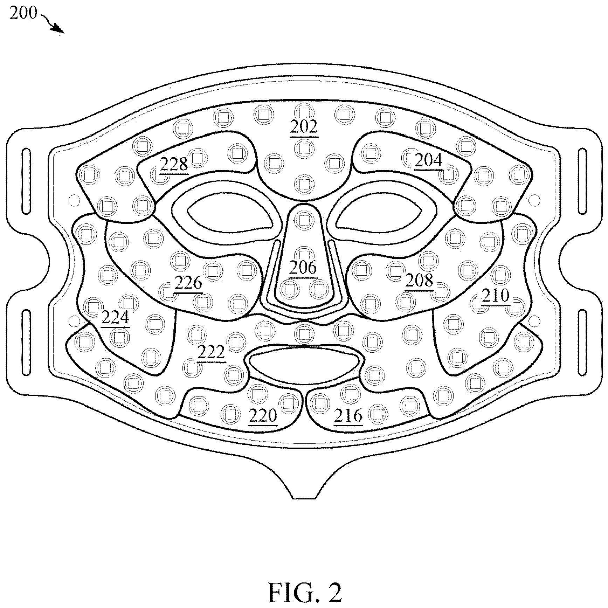

**FIG. 2** illustrates the predetermined distribution pattern **200** of the plurality of irradiation sources **116**, in accordance with an embodiment. The predetermined distribution pattern **200** includes a plurality of irradiation zones **202**, **204**, **206**, **208**, **210**, **216**, **220**, **222**, **224**, **226**, and **228.** In several embodiments of the invention, the plurality of irradiation zones **202**, **204**, **206**, **208**, **210**, **216**, **220**, **222**, **224**, **226**, and **228** may be divided into a first group of irradiation zones, a second group of irradiation zones, and a third group of irradiation zones. The first group of irradiation zones **202** and **222** may be configured to be around a forehead region and a mouth region of the facial region of the user. Furthermore, the irradiation sources in the first group of irradiation zones **202** and **222** may be configured to emit the electromagnetic radiation of wavelengths 625 - 640 nm, 655 - 665 nm, and 825 - 835 nm. Further, the second group of irradiation zones **204**, **208**, **226**, and **228** may be configured to be around two eyes of the facial region of the user. The irradiation sources in the second group of irradiation zones **204**, **208**, **226,** and **228** may be configured to emit the electromagnetic radiation of wavelengths 525 - 540 nm. The third group of irradiation zones **206**, **210**, **216**, **220**, and **224** may be configured to be around a nose region, a left side-cheek, a right side-cheek, and a jaw region of the facial region of the user. Also, irradiation sources in the third group of irradiation zones **206**, **210**, **216**, **220**, and **224** may be configured to emit electromagnetic radiation of wavelengths 585 - 595 nm.

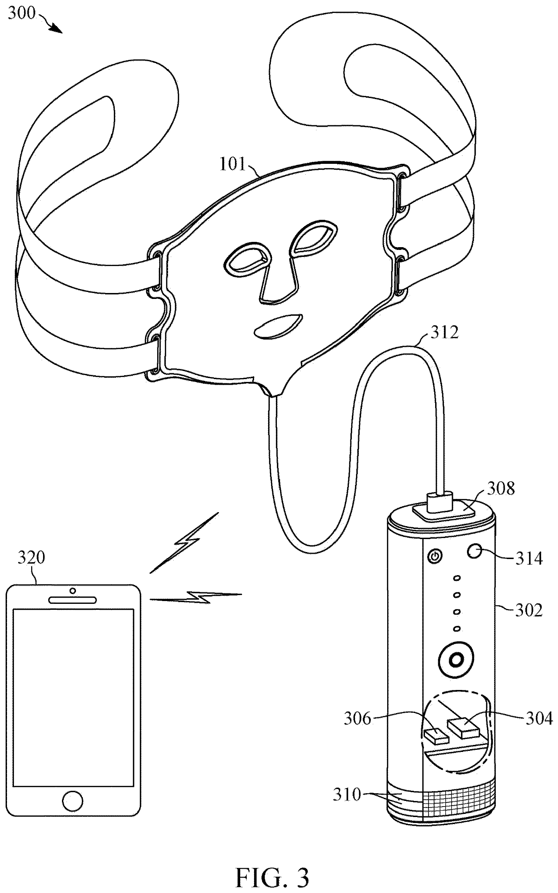

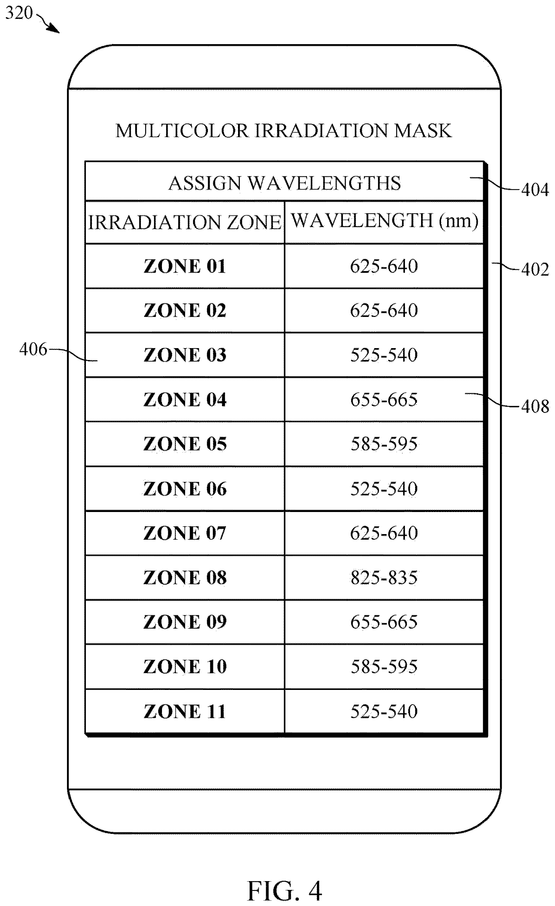

In several alternate embodiments of the invention, the plurality of irradiation sources **116** include RGB LEDs or monolithic multicolor lasers made from semiconductor materials. In that regard, the respective wavelengths of the electromagnetic radiation emitted by the irradiation sources in each one of the plurality of irradiation zones **202**, **204**, **206**, **208**, **210**, **216**, **220**, **222**, **224**, **226**, and **228** may be selected by a user and communicated to a control module of the facial irradiation device 100 through a user computing device **(*See*** ***FIGS 3*** ***and*** ***4*****)***.* The control module may receive a wavelength assignment scheme **(*See Table* 1)** including a plurality of respective wavelengths assigned to the plurality of irradiation zones **202**, **204**, **206**, **208**, **210**, **216**, **220**, **222**, **224**, **226**, and **228.**

**FIG. 3** illustrates a connection **300** between a control module **302** and the facial mask **101** of the facial irradiation device **100**, in accordance with an embodiment of the present invention. The connection **300** may be established using a Universal Serial Bus (USB) cable **312** or may be established wirelessly using Bluetooth, Zigbee, or Wi-Fi protocols. The facial irradiation device **100** further includes a power source **310** configured to provide electrical power for the facial irradiation device **100**. In that regard, it is envisaged that the power source **310** may include rechargeable batteries such as Lithium-Ion batteries, Lithium-Polymer batteries, and Nickel-Metal-Hydride batteries. Furthermore, the control module 302 includes an indicator 314 configured to indicate the remaining capacity of the power source 310 while in use. The indicator 314 may include a notification LED, a speaker, or an LCD or LED-based display unit.

The control module 302 further includes a processor 304 and a memory unit 306 operably connected to the processor 304. The memory unit 306 includes machine-readable instructions that when executed by the processor **304**, enable the processor **304** to control the supply of the electrical power to the plurality of irradiation sources **116**. In that manner, the plurality of irradiation sources **116** is configured to be operated in a plurality of operating modes. In several embodiments of the invention, the plurality of operating modes includes at least a first operating mode, a second operating mode, a third operating mode, and a fourth operating mode.

In the first operating mode, the irradiation sources in the first group of irradiation zones **202** and **222** are activated to emit the electromagnetic radiation with the wavelengths of 625 - 640 nm (Red), 655 - 665 nm (Deep Red), and 825 - 835 nm (Infrared). In the second operating mode, the irradiation sources in the first group of irradiation zones **202** and **222** are activated to emit the electromagnetic radiation with the wavelengths of 825 - 835 nm (Infrared) and the irradiation sources in the second group of irradiation zones **204**, **208**, **226**, and **228** are activated to emit the electromagnetic radiation with the wavelengths of 525 - 540 nm (Green). In the third operating mode, the irradiation sources in the third group of irradiation zones **206**, **210**, **216**, **220**, and **224** are activated to emit the electromagnetic radiation with the wavelengths of 585 - 595 nm (Yellow). Also, in the fourth operating mode, the irradiation sources in the first group of irradiation zones **202** and **222** are activated to emit the electromagnetic radiation with the wavelengths of 625 - 640 nm (Red), the irradiation sources in the second group of irradiation zones **204**, **208**, **226,** and **228** are activated to emit the electromagnetic radiation with the wavelengths of 525 - 540 nm (Green), and the irradiation sources in the third group of irradiation zones **206**, **210**, **216**, **220**, and **224** are activated to emit the electromagnetic radiation with the wavelengths of 585 - 595 nm (Yellow).

In several embodiments of the invention, the plurality of operating modes includes a fifth operating mode. In the fifth operating mode, the irradiation sources in the first group of irradiation zones **202** and **222** are activated to emit the electromagnetic radiation with the wavelengths of 625 - 640 nm (Red), 655 - 665 nm (Deep Red), and 825 - 835 nm (Infrared), the irradiation sources in the second group of irradiation zones **204**, **208**, **226**, and **228** are activated to emit the electromagnetic radiation with the wavelengths of 525 - 540 nm (Green), and the irradiation sources in the third group of irradiation zones **206**, **210**, **216**, **220**, and **224** are activated to emit the electromagnetic radiation with the wavelengths of 585 - 595 nm (Yellow).

The operating modes may be switched by the processor **304** based on pre-programming of the control module **302** or based on a control input received from a user computing device **320** via a communication interface **308** of the control module **302**. In several embodiments of the invention, the control input received by the processor 304 may include a wavelength assignment scheme corresponding to an operating mode. The processor 304 in that regard may control the supply of the electrical power to the plurality of irradiation sources based on the wavelength assignment scheme. In that regard, the wavelength assignment scheme may include a plurality of respective wavelengths assigned to the plurality of irradiation zones **202**, **204**, **206**, **208**, **210**, **216**, **220**, **222**, **224**, **226**, and **228**. **Table 1** below depicts an example wavelengths assignment scheme, in accordance with an embodiment of the present invention.

**Table 1**

| **IRRADIATION ZONE** | **ASSIGNED WAVELENGTH** |
|---|---|
| ZONE 01 (**202**) | 625 - 640 nm |
| ZONE 02 (**204**) | 625 - 640 nm |
| ZONE 02 (**206**) | 525 - 540 nm |
| ZONE 04 (**208**) | 655 - 665 nm |
| ZONE 05 (**210**) | 585 - 595 nm |
| ZONE 06 (**216**) | 525 - 540 nm |
| ZONE 07 (**220**) | 625 - 640 nm |
| ZONE 08 (**222**) | 825 - 835 nm |
| ZONE 09 **(224**) | 655 - 665 nm |
| ZONE 10 (**226**) | 585 - 595 nm |
| ZONE 11 (**228**) | 525 - 540 nm |

**FIG. 4** illustrates an example user interface **402** of the user computing device **320** for generating the wavelength assignment scheme, in accordance with an embodiment of the present invention. The user interface **402** includes a wavelength assignment scheme **404**. The wavelength assignment scheme **404** lists all the applicable zones in a first column **406** and allows a user to enter the intended respective wavelengths to be assigned in a second column **408**. The assigned wavelengths depicted in **FIG. 4** are identical to those listed in **Table 1**.

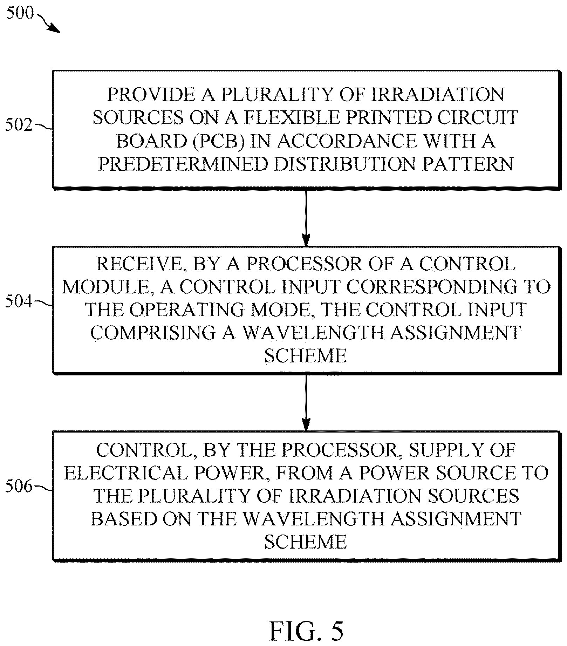

**FIG. 5** illustrates a method **500** for operating the facial irradiation device **100** in an operating mode of the plurality of operating modes, in accordance with an embodiment of the present invention. The method begins at Step **502** when the plurality of irradiation sources **116** are provided on the fourth surface **114** of the flexible PCB **110.** The plurality of irradiation sources **116** are provided on the fourth surface **114** in accordance with the predetermined distribution pattern **200.** The predetermined distribution pattern **200** includes a plurality of irradiation zones **202**, **204**, **206**, **208**, **210**, **216**, **220**, **222**, **224**, **226**, and **228.** The plurality of irradiation zones **202, 204, 206, 208**, **210**, **216**, **220**, **222**, **224**, **226**, and **228** may be divided into the first **202** and **222,** the second **204, 208**, **226**, and **228**, and the third **206, 210, 216, 220,** and **224** group of irradiation zones.

In several embodiments of the invention, the irradiation sources in the first group of irradiation zones **202** and **222** may be configured to emit electromagnetic radiation of wavelengths 625 - 640 nm, 655 - 665 nm, and 825 - 835 nm. Further, the irradiation sources in the second group of irradiation zones **204**, **208**, **226**, and **228** may be configured to emit electromagnetic radiation of wavelengths 525 - 540 nm. Also, the irradiation sources in the third group of irradiation zones **206**, **210**, **216**, **220**, and **224** may be configured to emit electromagnetic radiation of wavelengths 585 - 595 nm.

At Step **504**, the processor **304** receives the control input corresponding to the operating mode. The control input, in that regard, includes the wavelength assignment scheme including the plurality of respective wavelengths assigned to the plurality of irradiation zones **202**, **204**, **206**, **208**, **210**, **216**, **220**, **222**, **224**, **226**, and **228**. At Step 506, the processor 304 controls the supply of electrical power, from the power source **310** to the plurality of irradiation sources **116**, based on the wavelength assignment scheme.

Embodiments of the present invention, as presented above offer several advantages. For instance, red, deep red, and infrared radiations increase blood flow, encourage antioxidant activity, and lower inflammatory markers in the body. They also boost blood flow, bringing oxygen and nutrients to the skin cells to encourage healing and reduce inflammation. They also increase the production of collagen by stimulating collagen-producing cells called fibroblasts. Green light causes the small blood vessels that lie just beneath the skin to close down and disappear. Therefore, making their appearance less visible. Also, researchers indicate that green light is an effective treatment method against skin problems, such as rosacea and erythema, as it soothes the affected skin area. Yellow light is best known for skin cell rejuvenation and its soothing anti-inflammatory benefits. It also boosts lymphatic flow, which removes toxins and waste from the skin and promotes anti-aging.

Various modifications to these embodiments are apparent to those skilled in the art, from the description and the accompanying drawings. The principles associated with the various embodiments described herein may be applied to other embodiments. Therefore, the description is not intended to be limited to the embodiments shown along with the accompanying drawings but is to provide the broadest scope consistent with the principles and the novel and inventive features disclosed or suggested herein. Accordingly, the invention is anticipated to hold on to all other such alternatives, modifications, and variations that fall within the scope of the present invention and appended claims.

## Claims

1. A facial irradiation device, the facial irradiation device comprising:
a facial mask comprising:
an outer layer comprising a first surface facing towards the ambient and a second surface facing opposite to the first surface,
a flexible Printed Circuit Board (PCB) attached to the outer layer, the flexible PCB comprising a third surface facing towards the second surface and a fourth surface facing opposite to the third surface,
a plurality of irradiation sources attached to the flexible PCB at the fourth surface, and
an inner layer attached to the flexible PCB and/or the outer layer, the inner layer comprising a fifth surface facing towards the fourth surface and a sixth surface configured to be facing towards a facial region of a user;
a power source configured to provide electrical power; and
a control module comprising a processor and a memory unit operably connected to the processor, the memory unit comprising machine-readable instructions that when executed by the processor, enable the processor to:
control supply of the electrical power to the plurality of irradiation sources, such that, the plurality of irradiation sources is configured to be operated in a plurality of operating modes.

2. The facial irradiation device as claimed in claim 1, wherein the plurality of irradiation sources is selected from a group consisting of Light Emitting Diodes (LEDs) and lasers.

3. The facial irradiation device as claimed in claim 1, wherein the plurality of irradiation sources is configured to emit electromagnetic radiation in wavelengths of ranges comprising 525 - 540 nm, 585 - 595 nm, 625 - 640 nm, 655 - 665 nm, and 825 - 835 nm.

4. The facial irradiation device as claimed in claim 1, wherein the plurality of irradiation sources is attached to the fourth surface in accordance with a predetermined distribution pattern comprising a plurality of irradiation zones.

5. The facial irradiation device as claimed in claim 4, wherein for operating the plurality of irradiation sources in an operating mode of the plurality of operating modes, the processor is further enabled to receive a control input corresponding to the operating mode, from a user computing device, via a communication interface of the control module, the control input comprising a wavelength assignment scheme, the wavelength assignment scheme comprising a plurality of respective wavelengths assigned to the plurality of irradiation zones.

6. The facial irradiation device as claimed in claim 4, wherein the plurality of irradiation zones are divided into a first group of irradiation zones, a second group of irradiation zones, and a third group of irradiation zones, such that:
the first group of irradiation zones is configured to be around a forehead region and a mouth region of the facial region,
the second group of irradiation zones is configured to be around two eyes of the facial region, and
the third group of irradiation zones is configured to be around a nose region, a left side-cheek, a right side-cheek, and a jaw region of the facial region.

7. The facial irradiation device as claimed in claim 6, wherein:
irradiation sources in the first group of irradiation zones are configured to emit electromagnetic radiation of wavelengths 625 - 640 nm, 655 - 665 nm, and 825 - 835 nm;
irradiation sources in the second group of irradiation zones are configured to emit electromagnetic radiation of wavelengths 525 - 540 nm; and
irradiation sources in the third group of irradiation zones are configured to emit electromagnetic radiation of wavelengths 585 - 595 nm.

8. The facial irradiation device as claimed in claim 7, wherein the plurality of operating modes comprises:
a first operating mode wherein the irradiation sources in the first group of irradiation zones are activated to emit the electromagnetic radiation with the wavelengths of 625 - 640 nm, 655 - 665 nm, and 825 - 835 nm;
a second operating mode wherein the irradiation sources in the first group of irradiation zones are activated to emit the electromagnetic radiation with the wavelengths of 825 - 835 nm and the irradiation sources in the second group of irradiation zones are activated to emit the electromagnetic radiation with the wavelengths of 525 - 540 nm;
a third operating mode wherein the irradiation sources in the third group of irradiation zones are activated to emit the electromagnetic radiation with the wavelengths of 585 - 595 nm; and
a fourth operating mode wherein the irradiation sources in the first group of irradiation zones are activated to emit the electromagnetic radiation with the wavelengths of 625 - 640 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

9. The facial irradiation device as claimed in claim 8, wherein the plurality of operating modes comprises a fifth operating mode wherein the irradiation sources in the first group of irradiation zones are activated to emit the electromagnetic radiation with the wavelengths of 625 - 640 nm, 655 - 665 nm, and 825 - 835 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

10. The facial irradiation device as claimed in claim 8, wherein the processor is further enabled to switch between the plurality of operating modes based on a control input received from a user computing device via a communication interface of the control module.

11. The facial irradiation device as claimed in claim 1, wherein the power source includes one or more rechargeable batteries, and the control module comprises an indicator configured to indicate remaining capacity of the power source while in use.

12. The facial irradiation device as claimed in claim 1, wherein the second surface includes a plurality of protrusions configured to be in contact with the facial region of the user to maintain a predetermined gap between the inner layer and the facial region of the user.

13. The facial irradiation device as claimed in claim 1, wherein the inner layer is made up of a transparent silicone material.

14. A facial irradiation device, the facial irradiation device comprising:
a facial mask comprising:
an outer layer comprising a first surface facing towards the ambient and a second surface facing opposite to the first surface,
a flexible Printed Circuit Board (PCB) attached to the outer layer, the flexible PCB comprising a third surface facing towards the second surface and a fourth surface facing opposite to the third surface,
a plurality of irradiation sources attached to the flexible PCB at the fourth surface, and
an inner layer attached to the flexible PCB and/or the outer layer, the inner layer comprising a fifth surface facing towards the fourth surface and a sixth surface configured to be facing towards a facial region of a user;
a power source configured to provide electrical power; and
a control module comprising a processor and a memory unit operably connected to the processor, the memory unit comprising machine-readable instructions that when executed by the processor, enable the processor to:
control supply of the electrical power to the plurality of irradiation sources, such that, the plurality of irradiation sources is configured to be operated in a plurality of operating modes,
wherein the plurality of irradiation sources is attached to the fourth surface in accordance with a predetermined distribution pattern comprising a plurality of irradiation zones, and
wherein for operating the plurality of irradiation sources in an operating mode of the plurality of operating modes, the processor is further enabled to receive a control input corresponding to the operating mode, from a user computing device via a communication interface of the control module, the control input comprising a wavelength assignment scheme, the wavelength assignment scheme comprising a plurality of respective wavelengths assigned to the plurality of irradiation zones.

15. A method for operating a facial irradiation device in an operating mode of a plurality of operating modes, the method comprising:
providing a plurality of irradiation sources on a flexible Printed Circuit Board (PCB) in accordance with a predetermined distribution pattern, the predetermined distribution pattern comprising a plurality of irradiation zones;
receiving, by a processor of a control module, a control input corresponding to the operating mode, from a user computing device via a communication interface of the control module, the control input comprising a wavelength assignment scheme, the wavelength assignment scheme comprising a plurality of respective wavelengths assigned to the plurality of irradiation zones; and
controlling, by the processor, supply of electrical power, from a power source, to the plurality of irradiation sources based on the wavelength assignment scheme.

16. The method as claimed in claim 15, further comprising dividing the plurality of irradiation zones into a first group of irradiation zones, a second group of irradiation zones, and a third group of irradiation zones, such that:
the first group of irradiation zones is located around a forehead region and a mouth region of the facial region,
the second group of irradiation zones is located around two eyes of the facial region, and
the third group of irradiation zones is located around a nose region, a left side-cheek, a right side-cheek, and a jaw region of the facial region.

17. The method as claimed in claim 16, further comprising:
configuring irradiation sources in the first group of irradiation zones to emit electromagnetic radiation of wavelengths 625 - 640 nm, 655 - 665 nm, and 825 - 835 nm;
configuring irradiation sources in the second group of irradiation zones to emit electromagnetic radiation of wavelengths 525 - 540 nm; and
configuring irradiation sources in the third group of irradiation zones to emit electromagnetic radiation of wavelengths 585 - 595 nm.

18. The method as claimed in claim 17, wherein the plurality of operating modes comprises:
a first operating mode wherein the irradiation sources in the first group of irradiation zones are activated to emit the electromagnetic radiation with the wavelengths of 625 - 640 nm, 655 - 665 nm, and 825 - 835 nm;
a second operating mode wherein the irradiation sources in the first group of irradiation zones are activated to emit the electromagnetic radiation with the wavelengths of 825 - 835 nm and the irradiation sources in the second group of irradiation zones are activated to emit the electromagnetic radiation with the wavelengths of 525 - 540 nm;
a third operating mode wherein the irradiation sources in the third group of irradiation zones are activated to emit the electromagnetic radiation with the wavelengths of 585 - 595 nm; and
a fourth operating mode wherein the irradiation sources in the first group of irradiation zones are activated to emit the electromagnetic radiation with the wavelengths of 625 - 640 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

19. The method as claimed in claim 18, wherein the plurality of operating modes comprises a fifth operating mode wherein the irradiation sources in the first group of irradiation zones are activated to emit the electromagnetic radiation with the wavelengths of 625 - 640 nm, 655 - 665 nm, and 825 - 835 nm, the irradiation sources in the second group of irradiation zones are activated to emit the electromagnetic radiation with the wavelengths of 525 - 540 nm, and the irradiation sources in the third group of irradiation zones are activated to emit the electromagnetic radiation with the wavelengths of 585 - 595 nm.

20. The method as claimed in claim 18, further comprising switching, by the processor, between the plurality of operating modes based on the control input received from a user computing device via a communication interface of the control module.
